# EUROPEAN PATENT APPLICATION

(11) **EP 2 626 103 A1**
(43) Date of publication of application: **14.08.2013**
(21) Application number: 13154058.5
(22) Date of filing: 05.02.2013
(51) Int. Cl.: A61M 27/00, A61M 5/168

(54) **Flow control device**

(30) Priority: 08.02.2012 GB 201202145
(71) Applicant: CENTRAX LIMITED, Newton Abbot Devon TQ12 4SQ (GB)
(72) Inventor: Barr, Richard Henry Howard, Torquay, Devon TQ1 2HB (GB)
(74) Representative: Dowling, Andrew

(57) **Abstract**

A device 1 for diverting fluid from the ventricles of the brain to another area of the body comprises: an inlet 2; an outlet 4; and a resistance member 6, operatively connected to the inlet 2 and the outlet 4, the resistance member 6 comprising: a first plate 10, a surface of which comprises a groove which defines a resistance flow channel, an entry of the flow channel being in fluid communication with the inlet 2 and an exit of the flow channel being in fluid communication with the outlet 4; and a second plate 8, the second plate being held in abutment with the grooved surface of the first plate 10 so as to define a resistance tube 16. The resistance tube 16 comprises a plurality of arcs 17 arranged in flow series, each arc radially extending between the inner and outer diameters of the first and second plates. A selection means 40 operable to interconnect a selected number of the arcs to the inlet and outlet is provided.

## Description

The present invention relates to a flow control device for diverting fluid and particularly, although not exclusively, relates to a device for adjustably metering fluid from the ventricles of the brain to another part of the body.

### BACKGROUND

Hydrocephalus is a defective condition of the brain caused by an imbalance between production of cerebral spinal fluid (CSF) within the brain and the capacity of the brain to re-absorb such fluid at normal pressure. Hydrocephalus may be congenital, accidental or age related and can result in loss of a wide range of physical and mental faculties. The accepted method of treatment of the condition is to divert excess fluid which the brain, through its impairment, is unable to absorb, to some other part of the body such as the right atrium or the peritoneal cavity, where the fluid can re-enter the blood stream. The primary technical challenge associated with this method of treatment is developing the capacity to control the conditions of pressure and flow within the brain in such a manner as to enable lost faculties to be restored, depending on the severity of the condition. This challenge is exacerbated by the greatly differing range of impairment between different patients.

Various devices have been developed to control the pressure and flow conditions within the brain to desired levels. Such devices are commonly known as valves. If the flow imbalance referred to above is not addressed, pressure within the brain rises to an abnormal level. Such increased pressure causes the ventricles of the brain to expand and causes abnormal stress and damage within the brain tissue. Consequently, many valve devices focus on a technology that controls the pressure within the ventricles of the brain directly, restricting that pressure to a desired level and allowing flow rates to vary to accommodate the target pressure. Such devices are referred to as pressure control valves and commonly comprise an orifice that may be forced open by fluid pressure within the brain against some form of resistant mechanism such as a ball and spring or a slit in a tubular member. The majority of valves in use at the present time are pressure control valves. Difficulties with this type of valve arise in being able to set the spring pressure accurately. In addition, the valves are susceptible to malfunction when subject to pressure waves which occur in the brain and they are also very sensitive to changes in pressure due to body posture.

An alternative type of valve directly controls the amount of fluid diverted. Control is effected in such a manner that the desired pressure conditions within the brain are achieved as a secondary effect. Such devices are referred to as flow control valves. Currently available valves control flow by some form of restrictive orifice, which in some cases can be varied to give different flow rates. However, at the pressures existing in the brain (around 12cm H₂O in a normal supine person), and at the very low diverted flow rates involved (some fraction of 0.35ml/min) the size of orifice is minute, and it is difficult to predetermine accurately the resulting flow.

EP2155314discloses a device for diverting fluid from the ventricles of the brain to another area of the body, the device comprising: an inlet; an outlet; and a resistance member, operatively connected to the inlet and the outlet, the resistance member comprising: a first plate, a surface of which comprises a groove which defines a resistance flow channel, an entry of the flow channel being in fluid communication with the inlet and an exit of the flow channel being in fluid communication with the outlet; and a second plate, the second plate being held in abutment with the grooved surface of the first plate so as to define a resistance tube.

A resistance tube such as that provided by EP21553141imits flow through the tube by frictional resistance on the walls of the tube, and thereby provides highly predictable and stable control of pressure and flow within the brain of a patient. Due to the nature of the device, it is not substantially influenced by pressure waves within the fluid it diverts and it is far less sensitive to body posture than a pressure control valve.

The present invention seeks to provide a metering device having a greater degree of adjustability than is provided by the prior art devices. In one embodiment the flow control device provides adjustment in a sufficient number of steps to provide graduated fine adjustment in the manner of a rheostat.

### STATEMENTS OF INVENTION

According to the present invention there is provided a device adapted to divert fluid from a part of a human or animal body in a controlled manner, the device comprising:
an inlet;
an outlet; and
a resistance member, operatively connected to the inlet and the outlet, the resistance member comprising:
   a first plate portion, a surface of which comprises a groove;
   a second plate portion, a surface of which is held in abutment with the groove of the first plate portion so as to define a resistance flow channel extending between an inner diameter of the first and second plate portions and an outer diameter of the first and second plate portions, the flow channel comprising a plurality of arcs arranged in flow series, each arc radially extending between the inner and outer diameters of the first and second plate portions, and;
   a selection means operable to interconnect a selected number of the arcs to the inlet and outlet, thereby varying the effective length of the resistance flow channel progressively over the whole range of resistance defined by the total length of the resistance flow channel.

The arcs may comprise involute shaped arcs. The shape of the arcs may be such as to enable the full length of the resistance flow channel to occupy substantially the whole of the first and second plate portion surfaces.

A U-shaped bend in the groove may be provided between neighbouring arcs. The U-shaped bend may be provided at the inner diameter end and/or outer diameter end of the arcs.

The resistance member may comprise a plurality of exits along its length. Each exit may be placed in fluid communication with the outlet by the selection means so as to vary the effective length of the resistance member. By varying the effective length of the resistance member, it is possible to tailor the device to treat patients having greatly differing levels of impairment.

The exits of the resistance member may communicate with the outlet via the selection means. The selection means imparts an element of selectivity and flexibility to the device.

An exit may be provided for every arc, e.g. every arc may comprise an exit. An exit may be provided for every other arc, e.g. every other arc may comprise an exit. The exits may be provided at the inner diameter and/or outer diameter of the first and second plate portions. A greater degree of control and adaptability may be provided by providing each or every other arc with an exit. A smoother transition between exits may also be provided.

The exit may comprise an orifice in one of the first and second plate portions. The orifice may be covered or uncovered by the selection means.

The selection means may comprise a rotor, which may be mounted adjacent the resistance member. The rotor may have a through passage. The rotor may be mounted for rotation relative to the resistance member, such that an exit of the resistance member may be brought into fluid communication with the outlet via the through passage.

The rotor may comprise an arcuate groove in fluid communication with the through passage. The arcuate groove may be arranged to overlap with a selected exit and may have a length sufficient to ensure an overlap with an exit always occurs.

The rotor and second plate portion may be integral.

The device may further comprise at least one additional plate portion held in abutment with one of the first and second plate portions. A further groove may be provided on a surface at the interface between the additional plate portion and the one of the first and second plate portions. The further groove may define a further resistance flow channel. The further resistance flow channel may extend between the inner and outer diameters of the plate portions. The further resistance flow channel may comprise a plurality of arcs arranged in flow series. Each arc may radially extend between the inner and outer diameters of the plate portions. The further resistance flow channel may be in fluidic communication with the resistance flow channel with the arcs of the further resistance flow channel in alternating series with the arcs of the resistance flow channel. In other words, the further resistance flow channel and resistance flow channel may be intermeshed and successive arcs of the resistance flow channel may be arranged in an alternating fashion with arcs of the further resistance flow channel. The further resistance channel may provide additional resistance length and as such may add flexibility to the device without causing the device to increase in size significantly.

The further resistance flow channel may be in fluidic communication with the resistance flow channel through a plurality of openings in the first or second plate portions. The openings may fluidically connect the arcs of the further resistance flow channel in alternating series with the arcs of the resistance flow channel. The openings may correspond in location with the exits.

The first and/or second plate portions may be integral with a housing of the device. Alternatively, the first and/or second plate portions may be separate components, e.g. from the housing.

The surface of the first plate portion may be substantially annular. The surface of the second plate portion may be substantially annular. The surface of the additional plate portion may be substantially annular.

The device may be configured to be implanted under the skin of, for example, the scalp or chest of a person. It is an advantage of the device that it may be constructed in a highly compact fashion, accommodating the required length of resistance member within a relatively small surface area. The device can therefore be made sufficiently small and compact to be implanted under the skin of a patient.

The effective hydraulic mean diameter of the resistance member may be between 0.3 mm and 0.8mm. Optionally, the effective hydraulic mean diameter of the resistance member may be between 0.4 mm and 0.5 mm. For example, the effective hydraulic mean diameter of the resistance member may be 0.45 mm. The suggested ranges of effective hydraulic mean diameters ensure a desired flow rate is maintained within a device that is of a size suitable for implantation under the skin.

The resistance member may define a convoluted fluid flow path. For example, it may be folded back on itself at least once. Such a convoluted flow path enables the device to accommodate the high length to diameter ratio required for functionality whilst remaining compact and occupying a relatively small surface area.

The selection means may be magnetically excitable, such that it may be operated via a magnetic controller held proximate the device. The device may therefore be operated transcutaneously. Once implanted, the device may be adjusted to suit the changing needs of a patient in situ, without the need for further surgical intervention.

The selection means may be a sliding fit with the resistance member. Alternatively or in addition, the device may comprise a biasing means, which may resiliently urge the selection means into engagement with the resistance member.

The biasing means may comprise a spring, a Belleville washer or a resilient element.

The device may further comprise a biased ratchet or brake that releasably retains the selection means in a selected rotational orientation. The selection means may thus be securely held in its desired position when not being adjusted.

The selection means may further comprise a magnetic marker, such that the orientation of the selection means may be determined through use of a compass held proximate the device. Such a marker may assist in monitoring the operation of the device and in adjusting the device if necessary.

The device may further comprise a filter at the inlet of the device. The filter may reduce the possibility of the resistance member becoming occluded.

The device may further comprise an anti reflux mechanism at the outlet of the device. Such a mechanism may ensure that reflux back towards the origin of the fluid cannot take place for any reason.

The groove on the first plate may be formed in any appropriate manner. For example, the grove may be milled, laser cut, electro-discharge machined, electrochemically machined, chemically etched, or moulded. Such techniques may enable the resistance channel, and hence the resistance member, to be machined to the extremely high tolerances required to achieve accuracy of flow control.

The device may be formed from a biocompatible material selected for example from titanium, stainless steel alloy, or composite material.

The device may further comprise a fixing element that maintains the first and second plates in abutment. For example, the fixing element may comprise one of a screw, a weld, a glue joint, a brazed joint or a cap. Components of the device, such as the first and second plates, may be held together by virtue of an interference fit.

The inlet and the outlet may each define an axis and the axes of the inlet and outlet may or may not be parallel. The positioning of the inlet and outlet may facilitate routing of tubing to the area of the body where fluid is to be discharged.

According to another aspect of the present invention, there is provided a method of treating hydrocephalus by diverting fluid from the ventricles of the brain to another area of the body using the above-mentioned device.

### BRIEF DESCRIPTION OF THE DRAWINGS

For a better understanding of the present invention, and to show more clearly how it may be carried into effect, reference will now be made, by way of example, to the accompanying drawings, in which:-
Figure 1a is a sectional view of a first embodiment of a device for diverting fluid.
Figure 1b is a partially cut away sectional view of the device of Figure 1a, taken at 90 degrees to the view of Figure 1a.
Figure 1c is a perspective view of the rotor shown in Figures 1a and 1b.
Figure 2a is a sectional view of a second embodiment of a device for diverting fluid.
Figure 2b is a partially cut away sectional view of the device of Figure 2a, taken at 90 degrees to the view of Figure 2a.
Figure 3a is a sectional view of a third embodiment of a device for diverting fluid.
Figure 3b is a partially cut away sectional view of the device of Figure 3a, taken at 90 degrees to the view of Figure 3a.

### DETAILED DESCRIPTION OF PREFERRED EMBODIMENTS

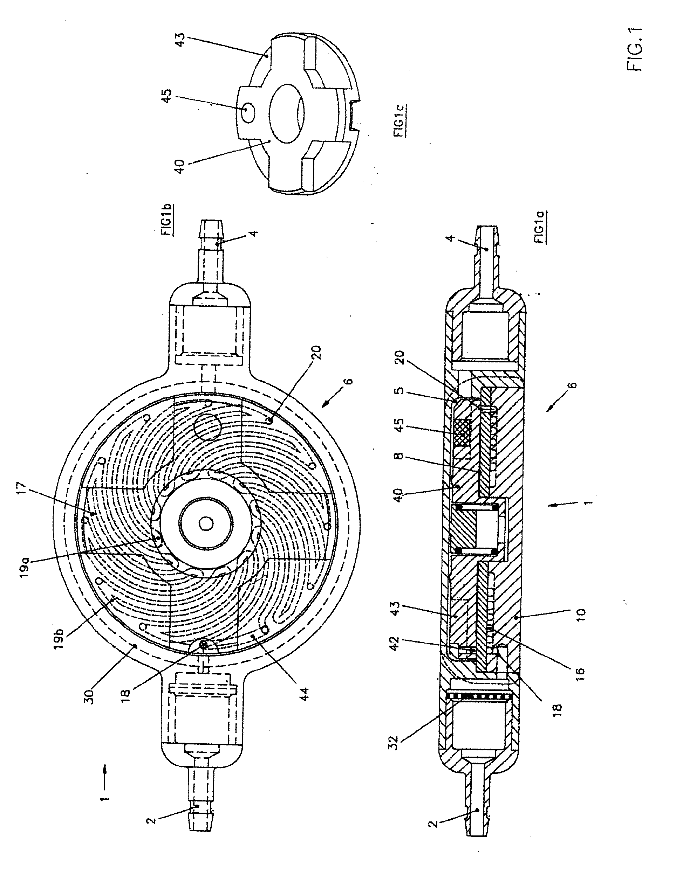

With reference to Figures 1a, 1b and 1c, a first embodiment of a device 1 for diverting fluid from the ventricles of the brain comprises an inlet 2, an outlet 4 and a resistance member 6 operatively coupled between the inlet 2 and the outlet 4. The resistance member 6 comprises an upper member 8 and a lower member 10. The upper and lower members 8, 10 each have substantially circular, planar surfaces which are in contact with each other. The upper and lower members 8, 10 may thus comprise or be referred to as first and second plate portions. The planar surfaces of the upper and/or lower members 8, 10 may comprise a groove or channel, which forms a continuous closed resistance flow channel or tube 16 when the planar surfaces are in abutment. However, in the embodiment shown in Figure 1, just the lower member 10 comprises such a groove. The resistance tube 16 follows a convoluted flow path from a fluid entry point 18, at a radially outer position on the planar surface of the lower member 10, to a plurality of fluid exit points 20, at radially outer positions on the planar surface of the lower member 10. The fluid entry point 18 of the resistance tube 16 is in fluid communication with the inlet 2 of the device 1.

The convoluted flow path of the resistance tube 16 extends between inner and outer diameters of the circular, planar surfaces of the upper and lower members 8, 10. The resistance tube 16 comprises a plurality of arcs 17 arranged in flow series, each arc radially extending between the inner and outer diameters of the upper and lower members 8, 10. The arcs 17 comprise substantially involute shaped arcs. For example, the arcs 17 extend radially whilst also traversing in a single circumferential direction. In other words, there may be no point of inflection between the ends of each radially extending arc 17. Each arc 17 traverses in the same circumferential direction and the arcs have substantially the same shape. The arcs 17 are equiangularly distributed about a centre of the upper and lower members 8, 10. Accordingly, the arcs are arranged side by side in a nesting arrangement. The illustrated convoluted flow paths of the resistance tube 16 comprises a series of arcs 17 with each arc folding back upon itself at the tip of each arc and thus meandering from a radially outer position to a radially inner position or vice versa. The shape of the arcs 17 is such as to enable the full length of the resistance flow channel to occupy substantially the whole of the annulus and thus to maximise the length of the resistance tube 16 for a given annulus area.

As depicted in Figure 1b, U-shaped bends in the groove defining the resistance tube 16 may be provided between neighbouring arcs 17. A U-shaped bend 19a may be provided at the radially inner ends of arcs 17 and a U-shaped bend 19b may be provided at the radially outer ends of arcs 17. Due to the circumferential slant of the arcs 17, the U-shaped bend may appear as a skewed "U". The U-shaped bends 19a, 19b may be provided between alternating adjacent pairs of arcs 17 such that a continuous resistance tube 16 is provided with the U-shaped bends joining successive arcs 17. However, a U-shaped bend may not be provided between one pair of adjacent arcs 17 at the fluid entry point 18 of the resistance tube 16, thereby ensuring that fluid flowing in the resistance tube 16 may not return to the fluid entry point. Furthermore, the exit points 20 are positioned so as to coincide with the radially outer U-shaped bends 19b between neighbouring arcs 17 at the outer diameter end. Each exit point 20 comprises an opening in the upper member 8.

Each exit point 20 of resistance tube 16 may be placed in fluid communication with the outlet 4 of the device 1 via a selection rotor 40. The selection rotor 40 is mounted for rotation about a centre point of the upper and lower members 8, 10 and comprises a planar face that is held in abutment with, and may be spring loaded against, the surface of the upper member 8 that is opposite the surface which faces the grooved surface of the lower member 10.

Four cavities 43 in the rotor are provided to produce a cruciform rotor configuration which can be magnetically activated to produce induced magnetic poles on each limb of the cruciform, thereby enabling a rotating torque to be exerted on the rotor when under the influence of an external magnetic controller with a similar number of four cruciform limbs. Although the number of four has been chosen in this particular example the number of rotor limbs could be higher or lower so long as they correspond to the number of limbs of the controller. The rotor and magnetic controller together form a magnetic coupling.

The rotor 40 comprises a through passage 42, which passage is in fluid communication with the outlet 4 via a chamber 5 within which the rotor 40 is mounted. A further passage may be provided in the rotor to ensure the free movement of fluid towards the outlet 4. The further passage may comprise a recess in the limb of the rotor and/or a notch in a radially outer corner of the limb.

The opening of the passage 42 on the planar face of the rotor 40 is at the same radial position as the exit points 20 of the resistance tube 16. The opening of the through passage 42 may therefore be brought into alignment with any of the exit points 20 of the resistance tube 16 merely by moving the rotor 40 to the appropriate rotational position. The remaining exit points 20 of the resistance tube are sealed off by the planar face of the rotor 40. The resistance tube 16 thus has a fixed entry point 18, that is in fluid communication with the inlet 2 of the device 1, and a series of possible exit points 20, each of which may be placed in fluid communication with the outlet 4 of the device 1. The effective length of the compound resistance tube, and hence the pressure and flow characteristics of the device 1, may therefore be varied by bringing different exit points 20 into fluid communication with the outlet 4.

In addition to passage 42, the rotor comprises an arcuate groove 44 in fluid communication with the through passage 42. The arcuate groove 44 is provided in the surface of the rotor 40 facing the upper member 8 and exits 20. The arcuate groove 44 is arranged to overlap with a selected exit 20. The arcuate groove 44 may furthermore have a length sufficient to ensure an overlap with an exit always occurs. For example, at certain rotational positions of the rotor the arcuate groove 44 may overlap with and thus be in fluid communication with two exits 20 and at other rotational positions of the rotor the arcuate groove 44 may overlap with one exit, thereby ensuring an overlap with at least one exit.

The rotor 40 is magnetically excitable, so that it may be moved to a different rotational position through the correct orientation of the magnetic controller brought into close proximity to the rotor 40. In addition, the rotor 40 may be magnetically identifiable by virtue of a small marker magnet 45 provided on the rotor 40, e.g. in one of the ribs between cavities 43. In addition, the position of the rotor 40 may be determined even when the rotor is obscured from view (for example when the device is implanted under the skin) by holding a compass adjacent the rotor. The rotational position of the rotor may be stabilised by a spring-loaded ratchet (not shown) operating between the rotor and the surrounding casing such that a substantial torsional magnetic force must be exerted on the rotor 40 to move it from a particular rotational position. Alternatively, it may be located simply by frictional forces caused by the spring loading.

The device 1 may comprise additional members (not shown) within the resistance member 6, each having a grooved surface in abutment with a planar surface in order to form a further length of resistance tube. In this manner a compound resistance tube of many layers may be provided without increasing the surface area occupied by the device 1.

The components of the resistance member 6 may be held together by laser welding, brazing, gluing, by virtue of friction or any other appropriate means of fixation. For example, the upper and lower members 8, 10 of the resistance member 6 may be maintained in abutment with one another by a single central screw. The lower member 10 of the resistance member 6 may be integrally formed with the inlet 2 and the outlet 4. A rim 30 extends around the circumference of the resistance member 6 and, together with the lower member 10, the inlet 2 and the outlet 4, forms a casing of the device 1 within which the remaining components are mounted.

A filter 32 is mounted in the inlet 2 of the device 1, in the fluid flow path from the inlet 2 to the entry 18 to the resistance tube 16. Although not shown, an anti-reflux mechanism may be mounted in the outlet 4 of the device 1, in the fluid flow path from the exit 20 of the resistance tube 16 to the outlet 4. The anti-reflux mechanism may be a non-return ball valve and may be spring-loaded. Alternatively, the mechanism may be any other suitable anti-reflux mechanism.

In the illustrated embodiment, the axes of the inlet 2 and outlet 4 are parallel. However, to facilitate routing of tubing leading from the outlet 4 to the area of the body selected for discharge of the fluid, it may be desirable for the axes of the inlet 2 and outlet 4 be angled with respect to one another.

In an advantageous application, the device 1 is mounted under the skin of a patient, but for tests or other purposes the device may be mounted externally, such as on the skin or even remote from the body, for example on clothing. CSF fluid from the brain enters the device 1 via the inlet 2 and passes through the filter 32. The filter 32 prevents occlusion of the resistance tube 16 by filtering from the CSF any particles of debris that might block the resistance tubes. Such debris may be particularly prevalent immediately after surgery. From the filter 32, CSF flows through the entry point 18 and into the resistance tube 16. CSF flows through the resistance tube 16 to the exit point 20 selected by the rotor 40. At the selected exit point 20, CSF passes through the arcuate groove 44, the through passage 42 and further passage in the rotor 40 and passes out of the device 1 via the outlet 4. If provided, the anti-reflux mechanism may prevent any reflux of CSF back towards the brain as a result of pressure changes caused by changes in body posture or for any other reason. The outlet 4 may be connected to appropriate tubing (not shown) to convey the CSF from the device 1 to the desired discharge area.

Frictional resistance from the walls of the resistance tube 16 limits CSF flow through the device to the desired flow rate. In order to achieve consistency of flow for a given length of resistance tube and pressure drop over the tube, the grooves on the lower member 10 that form the resistance tube 16 must be made to very close tolerances. From Poiseuille's equation, flow rate varies as the fourth power of channel diameter for a given length, pressure drop and fluid viscosity. Consequently, a small channel of approximately 0.5 mm effective diameter must be made to extremely close tolerances if the desired level of flow control is to be achieved. A preferred method of producing the grooves that form the resistance tubes, enabling the production of various contours over the length of the grooves, is CNC milling using a small end mill. This method of production is flexible, enabling the production of varying shapes and cross sections as desired. Other possible methods for forming the grooves include laser cutting, electro-discharge or electrochemical machining, chemical etching or moulding from a die if a suitable material is chosen. Suitable biocompatible materials for the device include titanium or stainless steel. Alternatively, a biocompatible composite would be required for moulding, for example High Density Polyethylene (HDPE), Ultra High Molecular Weight Polyethylene (UHMWPE) or Polyetheretherketone (PEEK).

Before implantation, the effective length of the resistance tube 16 required for a particular patient may be estimated and the rotor 40 moved to place the appropriate exit point 20 in fluid communication with the outlet 4. However, the pressure and flow characteristics of the device may be changed post operatively in a non-invasive manner by varying the rotational position of the rotor 40 and thus effective length of the resistance tube 16. The characteristics of the device could then be adapted according to postoperative observations of a patient. Accordingly, if, after implantation of the device 1, it is determined that the length of the resistance tube needs to be adjusted, in order to provide different pressure/flow characteristics, a compass is placed adjacent the skin covering the device 1 to determine the rotational orientation of the rotor 40. A magnetic controller is then brought into close proximity with the device and used to move the rotor 40 to the desired new rotational position. The provision of an exit point 20 at each radially outer U-bend 19b permits a greater degree of control and a smoother transition in the resistances obtained at each exit.

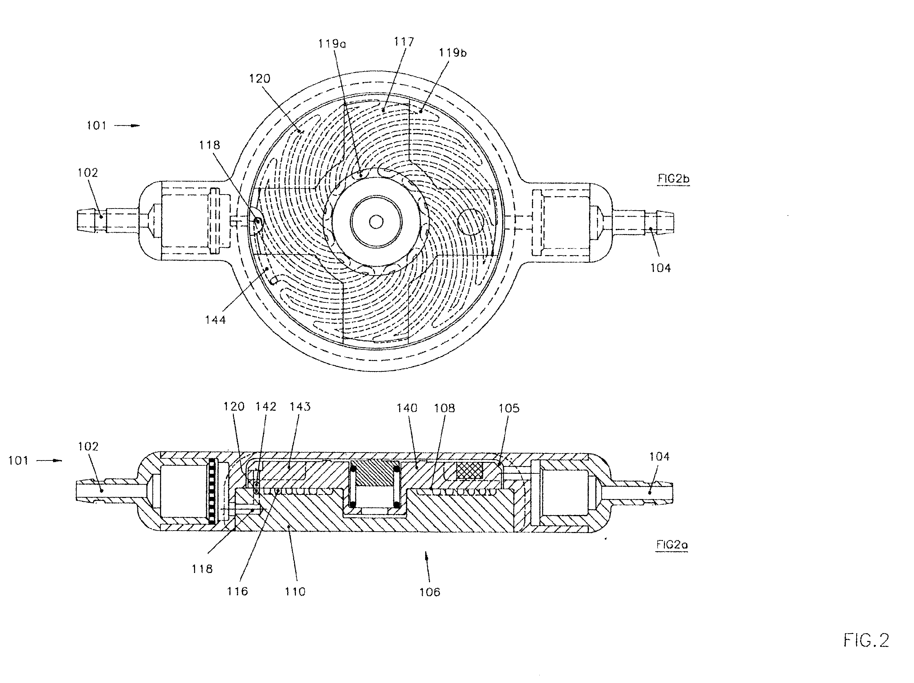

With reference to Figures 2a and 2b, a second embodiment of a device 101 is substantially similar in construction and operation to the device 1 of the first embodiment. As for the first embodiment, the device 101 is for diverting fluid from the ventricles of the brain and comprises an inlet 102, an outlet 104 and a resistance member 106 operatively coupled between the inlet 102 and the outlet 104. The resistance member 106 comprises an upper member 108 and a lower member 110. The upper and lower members 108, 110 each have substantially circular, planar surfaces which are in contact with each other. The upper and lower members 108, 110 may thus comprise or be referred to as first and second plate portions. The planar surface of the lower member 110 comprises a groove or channel, which forms a continuous closed resistance flow channel or tube 116 when the planar surfaces are in abutment. The resistance tube 116 follows a convoluted flow path from a fluid entry point 118, at a radially outer position on the planar surface of the lower member 110, to a plurality of fluid exit points 120, at a radially outer position on the planar surface of the lower member 110. The fluid entry point 118 of the resistance tube 116 is in fluid communication with the inlet 102 of the device 101.

The resistance tube 116 is arranged in a configuration equivalent to that of the resistance tube of the device 1 described above, with a corresponding plurality of arcs 117 arranged in flow series, each arc radially extending between the inner and outer diameters of the upper and lower members 108, 110. Again in a manner equivalent to that of the device 1 described above, U-shaped bends may be provided between neighbouring arcs 117 with U-shaped bends 119a provided at the radially inner ends of arcs 117 and U-shaped bends 119b provided at the radially outer ends of arcs 117. Fluid flowing through the device 101 therefore follows the same convoluted flow paths as previously described.

The device 101 also comprises a selection rotor 140 which may select an exit point of the resistance tube 16 to be placed in fluid communication with the outlet 4 of the device 1. The selection rotor 140 is mounted for rotation about a centre point of the lower member 110. However, in contrast to the device 1, the rotor 140 and upper member 108 are integral. Accordingly, the rotor 140 comprises a planar face that is held in abutment with, and may be spring loaded against, the grooved surface of the lower member 110.

Four cavities 143 in the rotor are provided to produce a cruciform rotor configuration which can be magnetically activated to produce induced magnetic poles on each limb of the cruciform, thereby enabling a rotating torque to be exerted on the rotor when under the influence of an external magnetic controller with a similar number of four cruciform limbs. Although the number of four has been chosen in this particular example the number of rotor limbs could be higher or lower so long as they correspond to the number of limbs of the controller. The rotor and magnetic controller together form a magnetic coupling.

The rotor 140 comprises a through passage 142, which passage is in fluid communication with the outlet 104 via a chamber 105 within which the rotor 140 is mounted. A further passage may be provided in the rotor to ensure the free movement of fluid towards the outlet 104. The further passage may comprise a recess in the limb of the rotor and/or a notch in a radially outer corner of the limb.

The opening of the passage 142 on the planar face of the rotor 140 is at the same radial position as exits of the resistance tube 116. The exits of the resistance tube may be defined by the radially outermost U-shaped bends 119b between the neighbouring arcs 117. The opening of the through passage 142 may therefore be brought into alignment with any of the U-shaped bends 119b of the resistance tube 116 merely by moving the rotor 140 to the appropriate rotational position. The remaining U-shaped bends 119b of the resistance tube are sealed off by the planar face of the rotor 140. The resistance tube 116 thus has a fixed entry point 118, that is in fluid communication with the inlet 102 of the device 101, and a series of possible exit points, each of which may be placed in fluid communication with the outlet 104 of the device 101. The effective length of the compound resistance tube, and hence the pressure and flow characteristics of the device 101, may therefore be varied by bringing different exit points into fluid communication with the outlet 104.

In addition to passage 142, the rotor comprises an arcuate groove 144 in fluid communication with the through passage. The arcuate groove 144 is provided in the surface of the rotor 140 facing the lower member 110. The arcuate groove 144 is arranged to overlap with a selected U-shaped bend 119b. The arcuate groove 144 may furthermore have a length sufficient to ensure an overlap with an exit always occurs. For example, at certain rotational positions of the rotor the arcuate groove 144 may overlap with and thus be in fluid communication with two U-shaped bends 119b and at other rotational positions of the rotor the arcuate groove 144 may overlap with one exit. Since the U-shaped bends 119b of the second embodiment may be circumferentially longer than the exit points 20 in the upper member 8 of the first embodiment, the arcuate groove 144 of the second embodiment may be smaller in length than the arcuate groove 44 of the first embodiment to ensure that there is always an overlap.

The device 101 according to the second embodiment is otherwise equivalent to the device 1 of the first embodiment and the features described with respect to the first embodiment may equally apply to the second embodiment. Accordingly, the operation of the device 101 is as described with respect to the device 1.

In an alternative arrangement to either of the first and second embodiments, the exits of the resistance tube 16, 116 may be provided at the radially innermost location or at an intermediate radial location. The opening of the passage 42, 142 on the planar face of the rotor 40, 140 may then be at the same radial position as the exits of the resistance tube 16, 116. For example, the exits 20, passage 42, 142 and arcuate groove 44, 144 may be provided at the radially innermost location, e.g. to coincide with the U-shaped bends 19a, 119a.

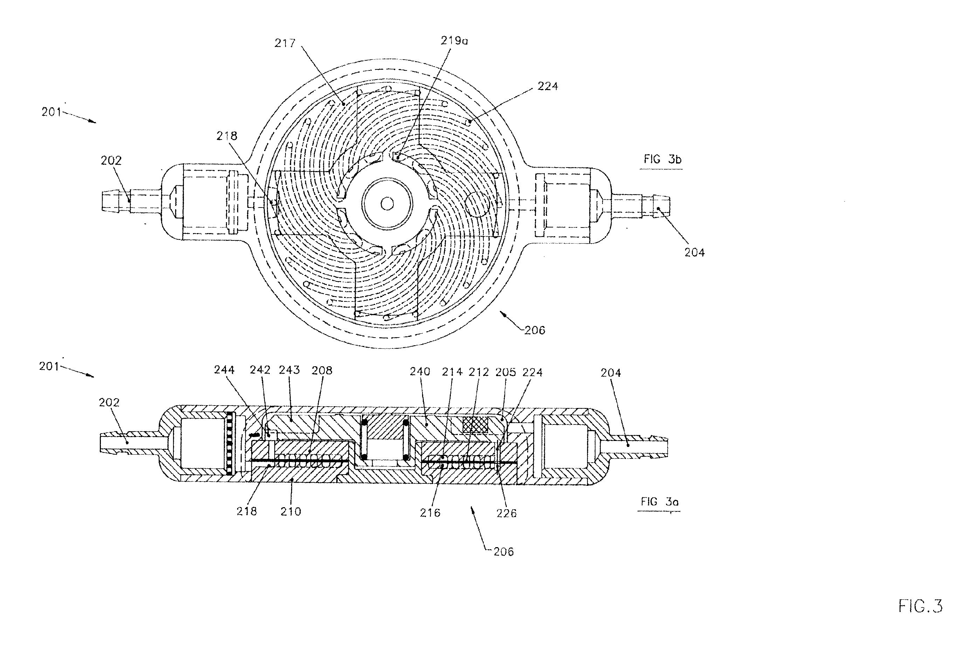

With reference to Figure 3a and 3b, a third embodiment of a device 201 is substantially similar in construction and operation to the device 1 of the first embodiment. As for the first embodiment, the device 201 is for diverting fluid from the ventricles of the brain and comprises an inlet 202, an outlet 204 and a resistance member 206 operatively coupled between the inlet 202 and the outlet 204. The resistance member 206 comprises an upper member 208, a lower member 210 and a substantially circular plate 212 sandwiched between the upper and lower members 208, 210. The upper and lower members 208, 210 each have a substantially circular, planar surface in contact with the plate 212. The plate 212 and upper and lower members 208, 210 may thus comprise or be referred to as plate portions. The planar surfaces of the upper and lower members 208, 210 each comprise a groove or channel, which forms a continuous closed resistance channel or tube 214, 216 when the grooved surface is in abutment with the plate 212. Alternatively, the grooves may be provided on a surface of the plate 212. The lower resistance tube 216 formed by the lower member 210 follows a convoluted flow path from a fluid entry point 218, at a radially outer position on the planar surface of the lower member 210. The fluid entry point 218 of the lower resistance tube 216 is in fluid communication with the inlet 202 of the device 201. The upper resistance tube 214 formed by the upper member 208 follows a convoluted flow path to a plurality of fluid exit points 224, at a radially outer position on the planar surface of the upper member 208. The fluid exit point 224 of the upper resistance tube 214 is in fluid communication with the outlet 204 of the device 201. The upper and lower resistance tubes 214, 216 are intermeshed with successive portions of the upper resistance tube 214 being arranged in an alternating fashion with portions of the lower resistance tube 216.

The convoluted flow path of the upper and lower resistance tubes 214, 216 extends between inner and outer diameters of the circular, planar surfaces of the upper and lower members 208, 210. The resistance tubes 214, 216 comprise a plurality of arcs 217 arranged in successive flow series, each arc radially extending between the inner and outer diameters of the upper and lower members 208, 210. The arcs 217 comprise substantially involute shaped arcs. For example, the arcs 217 extend radially whilst also traversing in a single circumferential direction. In other words, there may be no point of inflection between the ends of each radially extending arc 217. Each arc 217 traverses in the same circumferential direction and the arcs have substantially the same shape. The arcs 217 are equiangularly distributed about a centre of the upper and lower members 208, 210. Accordingly, the arcs are arranged side by side in a nesting arrangement. The illustrated convoluted flow paths of the resistance tubes 214, 216 comprise a series of arcs 217 with each arc joining a neighbouring arc at the radially innermost position and thus meandering from a radially outer position to a radially inner position and back to the radially outer position. The shape of the arcs 217 is such as to enable the full length of the resistance flow channel to occupy substantially the whole of the annulus and thus to maximise the length of the resistance tube 214, 216 for a given annulus area.

As depicted in Figure 3b, U-shaped bends may be provided between neighbouring arcs 217. A U-shaped bend 219a may be provided at the radially inner ends of arcs 217. Due to the circumferential slant of the arcs 217, the U-shaped bend may appear as a skewed "U". The U-shaped bends 219a may be provided between alternating adjacent pairs of arcs 217. However, in contrast to the first and second embodiments, U-shaped bends may not be provided at the radially outer ends for the third embodiment of the device 201. At the radially outer ends of the arcs 217, flow is instead transferred between the arcs 217 of the upper and lower resistance tubes 214, 216 by virtue of openings 216 in the plate 212. The upper resistance tube 214 is thus in fluidic communication with the lower resistance tube 216 through a plurality of openings 226 in the plate 212, the openings fluidically connecting the arcs of the upper resistance tube in alternating series with the arcs of the lower resistance tube. As a result, the upper resistance tube 214 is in fluidic communication with the lower resistance tube 216 with the arcs 217 of the upper resistance tube 214 in alternating series with the arcs 217 of the lower resistance tube 216. The openings 226 each comprise an orifice in the plate 212, permitting fluid communication between the two resistance tubes 214, 216 such that the two resistance tubes 214, 216 form a compound resistance tube having two superimposed layers. However, at the fluid entry point 218 of the lower resistance tube 216, an opening 226 may not be provided between an arc 217 in the upper member 208 and the adjacent arc 217 in the lower member 210. This would ensure that fluid flowing into the resistance tube 216 may not return to the fluid entry point and that the fluid may only flow in one direction through the resistance tubes 214, 216.

The exit points 224 may be positioned so as to coincide with the openings 226 between the upper and lower resistance tubes 214, 216. Each exit point 224 comprises an opening in the upper member 208.

Each exit point 224 of the upper resistance tube 214 may be placed in fluid communication with the outlet 204 of the device 201 via a selection rotor 240. The selection rotor 240 is mounted for rotation about a centre point of the upper and lower members 208, 210 and comprises a planar face that is held in abutment with, and may be spring loaded against, the surface of the upper member 208 that is opposite the surface which faces the plate 212.

Four cavities 243 in the rotor are provided to produce a cruciform rotor configuration which can be magnetically activated to produce induced magnetic poles on each limb of the cruciform, thereby enabling a rotating torque to be exerted on the rotor when under the influence of an external magnetic controller with a similar number of four cruciform limbs. Although the number of four has been chosen in this particular example the number of rotor limbs could be higher or lower so long as they correspond to the number of limbs of the controller. The rotor and magnetic controller together form a magnetic coupling.

The rotor 240 comprises a through passage 242, which passage is in fluid communication with the outlet 204 via a chamber 205 within which the rotor 240 is mounted. A further passage may be provided in the rotor to ensure the free movement of fluid towards the outlet 204. The further passage may comprise a recess in the limb of the rotor and/or a notch in a radially outer corner of the limb.

The opening of the passage 242 on the planar face of the rotor 240 is at the same radial position as the exit points 224 of the upper resistance tube 214. The opening of the through passage 242 may therefore be brought into alignment with any of the exit points 224 of the resistance tube 214 merely by moving the rotor 240 to the appropriate rotational position. The remaining exit points 224 of the resistance tube 214 are sealed off by the planar face of the rotor 240. The resistance tubes 214, 216 thus have a fixed entry point 218, that is in fluid communication with the inlet 202 of the device 201, and a series of possible exit points 224, each of which may be placed in fluid communication with the outlet 204 of the device 201. The effective length of the compound resistance tube, and hence the pressure and flow characteristics of the device 201, may therefore be varied by bringing different exit points 224 into fluid communication with the outlet 204.

In addition to passage 242, the rotor comprises an arcuate groove 244 in fluid communication with the through passage 242. The arcuate groove 244 is provided in the surface of the rotor 240 facing the upper member 208 and exits 224. The arcuate groove 244 is arranged to overlap with a selected exit 224. The arcuate groove 244 may furthermore have a length sufficient to ensure an overlap with an exit always occurs. For example, at certain rotational positions of the rotor the arcuate groove 244 may overlap with and thus be in fluid communication with two exits 224 and at other rotational positions of the rotor the arcuate groove 244 may overlap with one exit.

The device 201 according to the third embodiment is otherwise equivalent to the device 1, 101 of the first and second embodiments and the features described with respect to the first and second embodiments may equally apply to the third embodiment. Accordingly, the operation of the device 201 is as described with respect to the device 1, 101.

In alternative arrangements (not shown) the exit points from the resistance member may be provided at a radially inner position, the openings in the plate may also be placed at a radially inner position and/or the U-bends between neighbouring arcs may be provided at a radially outer position.

## Claims

1. A device adapted to divert fluid from a part of a human or animal body in a controlled manner, the device comprising:
an inlet;
an outlet; and
a resistance member, operatively connected to the inlet and the outlet, the resistance member comprising:
a first plate portion, a surface of which comprises a groove;
a second plate portion, a surface of which is held in abutment with the groove of the first plate portion so as to define a resistance flow channel extending between an inner diameter of the first and second plate portions and an outer diameter of the first and second plate portions, the flow channel comprising a plurality of arcs arranged in flow series, each arc radially extending between the inner and outer diameters of the first and second plate portions, and;
a selection means operable to interconnect a selected number of the arcs to the inlet and outlet, thereby varying the effective length of the resistance flow channel progressively over the whole range of resistance defined by the total length of the resistance flow channel.

2. A device as claimed in claim 1, wherein the arcs comprise involute shaped arcs.

3. A device as claimed in claim 1 or 2, wherein the shape of the arcs is such as to enable the full length of the resistance flow channel to occupy substantially the whole of the first and second plate portion surfaces.

4. A device as claimed in any of the preceding claims, wherein a U-shaped bend in the groove is provided between neighbouring arcs.

5. A device as claimed in claim 4, wherein the U-shaped bend is provided at the inner diameter end and/or outer diameter end of the arcs.

6. A device as claimed in any of the preceding claims, wherein the resistance member comprises a plurality of exits along its length, each of which may be placed in fluid communication with the outlet by the selection means so as to vary the effective length of the resistance member.

7. A device as claimed in claim 6, wherein an exit is provided for every arc.

8. A device as claimed in claim 6, wherein an exit is provided for every other arc.

9. A device as claimed in any of claims 6 to 8, wherein the exits are provided at the inner diameter and/or outer diameter of the first and second plate portions.

10. A device as claimed in any of claims 6 to 9, wherein the exit comprises an orifice in one of the first and second plate portions, which orifice may be covered or uncovered by the selection means.

11. A device as claimed in any of the preceding claims, wherein the selection means comprises a rotor mounted adjacent the resistance member and having a through passage, the rotor being mounted for rotation relative to the resistance member, such that an exit of the resistance member may be brought into fluid communication with the outlet via the through passage.

12. A device as claimed in claim 11, wherein the rotor comprises an arcuate groove in fluid communication with the through passage, the arcuate groove being arranged to overlap with a selected exit and having a length sufficient to ensure an overlap with an exit always occurs.

13. A device as claimed in claim 11 or 12, wherein the rotor and second plate portion are integral.

14. A device as claimed in any of the preceding claims, further comprising at least one additional plate portion held in abutment with one of the first and second plate portions,
wherein a further groove is provided on a surface at the interface between the additional plate portion and the one of the first and second plate portions, the further groove defining a further resistance flow channel,
wherein the further resistance flow channel extends between the inner and outer diameters of the plate portions, the further resistance flow channel comprising a plurality of arcs arranged in flow series, each arc radially extending between the inner and outer diameters of the plate portions, and
wherein the further resistance flow channel is in fluidic communication with the resistance flow channel with the arcs of the further resistance flow channel in alternating series with the arcs of the resistance flow channel.

15. A device as claimed in claim 14, wherein the further resistance flow channel is in fluidic communication with the resistance flow channel through a plurality of openings in the first or second plate portions, the openings fluidically connecting the arcs of the further resistance flow channel in alternating series with the arcs of the resistance flow channel.
